(19) Europäisches Patentamt
European Patent Office
Office européen des brevets

(11) Veröffentlichungsnummer: **0 071 138**
**A2**

# (12) EUROPÄISCHE PATENTANMELDUNG

(21) Anmeldenummer: 82106475.5

(22) Anmeldetag: 19.07.82

(51) Int. Cl.³: C 07 C 149/437
C 07 D 251/34, C 08 G 18/77
C 08 G 18/10, C 08 G 18/83
C 08 G 18/50

(30) Priorität: 29.07.81 DE 3129978

(43) Veröffentlichungstag der Anmeldung:
09.02.83 Patentblatt 83/6

(84) Benannte Vertragsstaaten:
BE DE FR GB IT

(71) Anmelder: BAYER AG
Zentralbereich Patente, Marken und Lizenzen
D-5090 Leverkusen 1, Bayerwerk(DE)

(72) Erfinder: Rasshofer, Werner, Dr.
Wolfskaul 10
D-5000 Köln(DE)

(72) Erfinder: Grögler, Gerhard, Dr.
von Diergardtstrasse 46
D-5090 Leverkusen(DE)

(72) Erfinder: Hess, Heinrich, Dr.
Auf der Griesse 48
D-5090 Leverkusen 1(DE)

(54) **Neue aromatische und aliphatische, schwefelhaltige primäre Amine, ein Verfahren zu ihrer Herstellung und ihre Verwendung zur Herstellung von Polyurethanen.**

(57) Die Erfindung betrifft neue, niedrig schmelzende oder bei Raumtemperatur flüssige, leicht lösliche, schwefelhaltige primäre aliphatische und aromatische Polyamine der Struktur $[H_2N-A-]_n \cdot B$, worin n = 2, 3 und 4 bedeutet, B für Harnstoff-, Biuret- und Isocyanuratreste oder den Rest

$G(OC.NH-)_n$,
||
O

worin G einen Rest einer n-wertigen Polyhydroxylverbindung mit dem Molekulargewicht 62 bis 10 000 bedeutet, steht und A für einen aromatischen Rest

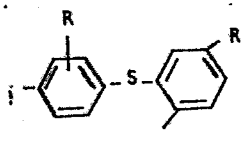

oder

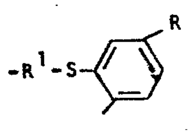

steht, bei dem R $C_1$-$C_4$-Alkyl-oder -Thioalkylgruppen und $R^1$ lineare oder verzweigte, aliphatische Kohlenwasserstoffreste mit 2-12 Kohlenstoffatomen sind.

Es wird auch ein Verfahren zur Hydrolyse entsprechender NCO-Voraddukte $(OCH-A)_nB$ mittels ausgewählter Basen, vzw. Alkali- oder Erdalkalihydroxide, über die Carbamatstufe und die Freisetzung der Amine durch Säurezugabe beansprucht. Ferner wird die Verwendung der neuen schwefelhaltigen Amine zur Herstellung von Polyurethanen beansprucht, wobei die flüssigen, löslichen Polyamine vorteilhaft eingesetzt werden können.

EP 0 071 138 A2

0071138

BAYER AKTIENGESELLSCHAFT
Zentralbereich
Patente, Marken und Lizenzen

5090 Leverkusen, Bayerwerk
OER/bc/c

# Neue aromatische und aliphatische, schwefelhaltige primäre Amine, ein Verfahren zu ihrer Herstellung und ihre Verwendung zur Herstellung von Polyurethanen

Die Erfindung betrifft neue, niedrigschmelzende oder bei Raumtemperatur flüssige, leicht lösliche schwefelhaltige primäre aliphatische und aromatische Polyamine, ein Verfahren zu ihrer Herstellung und ihre Verwendung zur Herstellung von Polyurethanen.

Aromatische Amine werden in großem Maße zur Vernetzung in PU-Kunststoffen verwendet. Eingesetzt werden z.B. 3,3'-Dichloro-4,4'-diaminodiphenylmethan ("Moca", Schmp. 110°C) oder 4-Chlor-3,5-diaminobenzoesäure-i-butylester (Schmp. 90°C). Weitere Beispiele für solche ein- und zweikernigen Polyaminoverbindungen finden sich in Angew. Makromol. Chemie, <u>26</u>, 29-45 (1972) und in der Firmenschrift der Ihara Chemical Industry Co., Ltd. "An overview of Polyurethane Diamino Curing Agents", verfaßt von W. Koike, T. Harada, H. Okashi und Ch. Yazawa.

Alle diese aromatischen Amine sind feste, kristalline Verbindungen mit z.T. hohen Schmelzpunkten. Aus diesen hohen Schmelzpunkten rühren verarbeitungstechnische

Nachteile. So muß zur Herstellung von PU-Elastomeren unter Verwendung von "Moca" dieses geschmolzen und als Schmelze mit flüssigen NCO-Prepolymeren homogenisiert werden. Dadurch werden aufwendige Absaugvorrichtungen notwendig, da sich Moca neben anderen als Vernetzer verwandten Diaminen wie z.B. Benzidin und 3,3'-Dichlorbenzidin im Tierversuch als cancerogen erwiesen hat.

Besonders nachteilig ist das Schmelzverhalten, wenn z.B. Harnstoffgruppen im Molekül anwesend sind. Solche Polyaminoharnstoffe, wie sie z.B. aus 2,4-Toluylendiamin und Phosgen im molaren Verhältnis 2:1 zugänglich sind, schmelzen entweder überhaupt nicht oder nur bei sehr hohen Temperaturen, so daß bei ihrer Verwendung heterogen gearbeitet werden muß, was - wie dem Fachmann bekannt ist - eine ganze Reihe von verarbeitungstechnischen Schwierigkeiten bereitet.

Der Hauptnachteil dieser Harnstoffgruppen aufweisenden Diamine des Standes der Technik ist darin zu sehen, daß sie bei dem genannten Verfahren zu ihrer Herstellung stets als nicht oder nur bei sehr hohen Temperaturen schmelzbare Festkörper der unterschiedlichsten Korngröße anfallen, so daß sie für ihre weitere Verarbeitung, beispielsweise als Ausgangsmaterial für die Herstellung von Polyurethanen nach ihrer Isolierung durch Filtration und Entfernung von anhaftenden Lösungsmittelmengen im Vakuum durch aufwendige Mahlvorgänge in eine feinverteilte Form gebracht werden müssen. Darüber hinaus werden bei der Umsetzung

dieser Harnstoffgruppen aufweisenden Diamine des Standes der Technik wegen ihres hohen Schmelzpunkts und ihrer Schwerlöslichkeit inhomogene Produkte erhalten, die im übrigen oft ein schlechtes mechanisches Eigenschaftsniveau aufweisen. Infolge des festen Aggregatzustandes und der Schwerlöslichkeit der Harnstoffgruppen aufweisenden Diamine des Standes der Technik sind bei ihrer Verarbeitung genaue Äquivalentverhältnisse zwischen Isocyanatgruppen und gegenüber Isocyanatgruppen reaktionsfähigen Gruppen nur schwer einzuhalten, da die Harnstoffdiaminteilchen oft nur an der Oberfläche abreagieren, wobei eine Hülle um nicht abreagiertes Harnstoffdiamin gebildet wird.

Andererseits stellen die Harnstoffgruppen aufweisenden Diamine wertvolle Ausgangsmaterialien zur Herstellung von Polyurethankunststoffen dar, da die durch ihre Verwendung als Aufbaukomponente eingebauten Harnstoffsegmente oft zu einer Verbesserung der mechanischen Eigenschaften des Kunststoffs führen.

Es war daher die Aufgabe der vorliegenden Erfindung, neue Harnstoffdiamine und ein Verfahren zu ihrer Herstellung zur Verfügung zu stellen, die einerseits wegen ihrer Harnstoffsegmente die Herstellung von Polyurethankunststoffen mit verbesserten mechanischen Eigenschaften gestatten, und die andererseits nicht mehr mit den geschilderten Nachteilen der Verbindungen bzw. Verfahren des Standes der Technik behaftet sind.

Es ist aber allgemein erwünscht, aus Verarbeitungsgründen möglichst niederviskose Ausgangskomponenten für die Polyurethan-Kunststoffherstellung zur Verfügung zu haben. Dies gilt auch für Aminogruppen enthaltende Ausgangsverbindungen. Es war daher auch die Aufgabe der vorliegenden Erfindung, neue, Urethan- und/oder Biuret- und/oder Isocyanuratgruppen enthaltende Polyamine zur Verfügung zu stellen, die sich gegenüber den Aminen des Standes der Technik durch eine niedrigere Viskosität und/oder bessere Löslichkeit in Polyhydroxylverbindungen sowie möglichst niedrigschmelzend oder flüssig sind, auszeichnen.

Diese Aufgaben konnten überraschenderweise dadurch gelöst werden, daß man bestimmte, nachstehend näher beschriebene, Schwefel aufweisende Diisocyanate, die Harnstoff- und/oder Biuret- und/oder Isocyanurat-, insbesondere aber Urethangruppen besitzen, mit "Basen" in die entsprechenden Carbamate überführt und diese Carbamate acidolytisch verseift. Als Basen im Sinne der Erfindung werden Oxide und Hydroxide aus der I., II. und II. Hauptgruppe des Periodensystems und/oder quartäre Ammoniumhydroxide verstanden.

Es ist bekannt, daß aromatische Isocyanate durch saure Hydrolyse in primäre Amine überführt werden

können. Die Reaktion verläuft allerdings nur sehr unvollständig, da das bei der Hydrolyse gebildete Amin mit noch nicht umgesetztem Isocyanat zum entsprechenden Harnstoff weiter reagiert. Diese Folgereaktion läßt sich auch durch Anwendung überschüssiger starker Mineralsäure nicht unterdrücken. Ein neueres Beispiel findet sich in der JP-PS 55007-827.

In der DE-B 1 270 046 wird ein Verfahren zur Herstellung definierter, Polyalkylenglykolether-Segmente enthaltender, primärer aromatischer Amine beschrieben, bei dem man Umsetzungsprodukte von aromatischen Di- oder Triisocyanaten mit Polyalkylenglykolethern und/ oder Polyalkylenglykolthioethern, vorzugsweise solchen mit Molekulargewichten zwischen 400 und 4000, mit sekundären oder tertiären Carbinolen umsetzt und anschließend (gegebenenfalls in Gegenwart saurer Katalysatoren) in einem inerten Lösungsmittel einer thermischen Spaltung unterwirft. Nachteilig ist dabei, daß im Verlauf der thermischen Spaltung der Urethane brennbare, leicht flüchtige Alkene entstehen, die im Gemisch mit Luft explosiv sind, so daß entsprechende Vorsichtsmaßnahmen getroffen werden müssen.

Gegenstand der DE-B 1 694 152 ist die Herstellung von mindestens zwei endständige Aminogruppen aufweisenden Präpolymeren durch Umsetzung von Hydrazin, Aminophenylethylamin oder anderen Diaminen mit einem NCO-Präpolymer aus einem Polyetherpolyol und Polyisocyanat (NCO:NH-Verhältnis = 1:1,5 bis 1:5). Nichtumgesetztes Amin muß dabei in einem weiteren Schritt

sorgfältig entfernt werden, da es die Umsetzung mit Polyisocyanaten stark katalysiert, so zu kurzen Verarbeitungszeiten führt und auch selbst als Reaktionspartner auftritt.

Eine andere Synthesemöglichkeit für Urethangruppen aufweisende Polyamine wird in der FR-PS 1 415 317 beschrieben. Urethangruppenhaltige NCO-Präpolymere werden mit Ameisensäure in die N-Formylderivate überführt, die zu endständigen aromatischen Aminen verseift werden. Auch die Reaktion von NCO-Präpolymeren mit Sulfaminsäure gemäß DE-B 1 555 907 führt zu Verbindungen mit endständigen Aminogruppen. Desweiteren werden höhermolekulare, aliphatische, sekundäre und primäre Aminogruppen aufweisende Voraddukte nach DE-B 1 215 373 durch Umsetzung höhermolekularer Hydroxylverbindungen mit Ammoniak in Gegenwart von Katalysatoren unter Druck bei hohen Temperaturen erhalten oder gemäß US-PS 3 044 989 durch Umsetzung höhermolekularer Polyhydroxylverbindungen mit Acrylnitril unter anschließender katalytischer Hydrierung. Auch durch Umsetzung von NCO-Präpolymeren mit Hydroxylgruppen aufweisenden Enaminen, Aldiminen oder Ketiminen und anschließende Hydrolyse erhält man gemäß DE-A 2 546 536 bzw. US-PS 3 865 791 höhermolekulare, endständige Aminogruppen und Urethangruppen aufweisende Verbindungen.

Gegenstand der vorliegenden Erfindung sind schwefelhaltige, niedrigschmelzende oder flüssige, leicht lösliche aromatische oder aliphatische Polyamine

der allgemeinen Formel $[H_2N-A]_n$ B, worin

n eine ganze Zahl von 2 bis 4 bedeutet,

B für n-wertige Reste der Strukturen

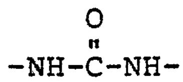 ,
(n=2)

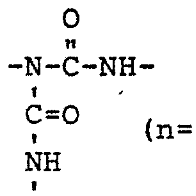
(n=3)
(Biuret-Rest)

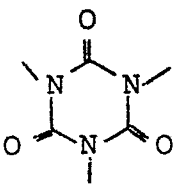
(n=3)
(Isocyanurat-Rest)

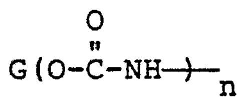 (n=2-4);

G für einen Rest, wie er durch Entfernung von n OH-Gruppen aus n-wertigen Polyhydroxylverbindungen des Molekulargewichtsbereichs 62-10 000 erhalten wird, und

A für einen aromatischen Rest der allgemeinen Formel

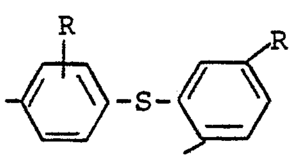

oder einen araliphatischen Rest der allgemeinen Formel

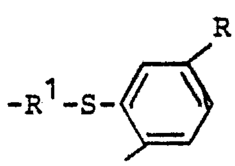

worin

R einen Alkyl- und/oder Thioalkylrest mit vorzugsweise 1-4 C-Atomen und

$R^1$ einen linearen oder verzweigten, aliphatischen Kohlenwasserstoffrest mit 2-12 Kohlenstoffatomen bedeuten, stehen.

Gegenstand der Erfindung ist ferner ein Verfahren zur Herstellung Schwefel enthaltender, gut löslicher, niedrig schmelzender oder flüssiger, niedrigviskoser, primärer, aromatischer oder aliphatischer Polyamine der allgemeinen Formel

$$[H_2N-A]_n\ B$$

worin

A,B und n wie oben beschrieben sind,

durch Vermischen von NCO-Voraddukten mit Basen aus der I., II. und III. Hauptgruppe des Periodensystems und/oder quartären Ammoniumhydroxiden in Gegenwart von Wasser, vorzugsweise wäßrigen Lösungen von Alkali- und Erdalkalihydroxiden, unter Bildung des Carbamats, Überführung des Carbamats durch Zusatz einer zur $OH^{\ominus}$-Menge mindestens geringfügig überschüssigen Menge eines Protondonators unter $CO_2$-Abspaltung in das freie Amin bzw. sein Salz und gegebenenfalls Freisetzung des Amins durch Basen dadurch gekennzeichnet, daß man ein Urethan- und/oder Harnstoff- und/oder Biuret- und/oder Isocyanuratgruppen aufweisendes NCO-Voraddukt der allgemeinen Formel

$$[OCN-A]_n\ B$$

worin

n eine ganze Zahl von 2 bis 4 bedeutet,

B für n-wertige Reste der Strukturen

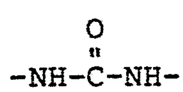 , 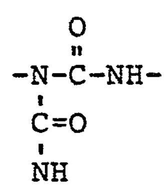

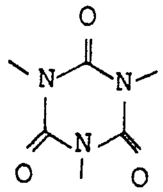

, $G(O-\overset{O}{\overset{\|}{C}}-NH-)_n$ ;

G für einen Rest, wie er durch Entfernen von n OH-Gruppen aus einer n-wertigen Polyhydroxylverbindung des Molekulargewichtsbereiches 62-10 000 erhalten wird, und

A für einen aromatischen Rest der allgemeinen Formel

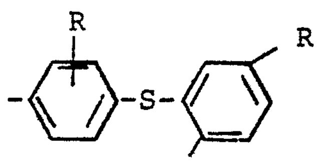

oder einen araliphatischen Rest der allgemeinen Formel

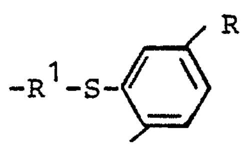

worin

R einen Alkyl- und/oder Alkylthiorest mit 1 bis 4 Kohlenstoffatomen und

$R^1$ einen linearen oder verzweigten, aliphatischen Kohlenwasserstoffrest mit 2-12 Kohlenstoffatomen bedeuten, stehen,

verwendet.

1.

Zur Herstellung der neuen Polyamine $[H_2N-A]_n$ B wird zunächst ein Urethan- und/oder Harnstoff- und/oder Biuret- und/oder Isocyanuratgruppen aufweisendes NCO-Voraddukt der allgemeinen Formel

$$[OCN-A]_n\ B$$

worin

n,B,A,G,R und $R^1$ die oben angeführte Bedeutung haben,

gegebenenfalls gelöst in einem mit Wasser mischbaren, NCO-inerten organischen Lösungsmittel, bei einer Temperatur von ca. 0 bis 40°C, bevorzugt 10 bis 20°C, durch Vermischen mit Basen aus der Reihe der I., II. und III. Hauptgruppe des Periodensystems und/oder quartären Ammoniumhydroxiden, vorzugsweise einer wäßrigen Alkali- oder Erdalkalihydroxidlösung, in das entsprechende Carbamat überführt, wobei das Äquivalentverhältnis zwischen Hydroxid- und NCO-Gruppen z.B. 0,3 bis 1,01:1 ist und vorzugsweise zwischen 1,01:1 und 1,80:1 liegt;

2.

das gebildete Carbamat in einem zweiten Schritt durch Zusatz einer der $OH^{\ominus}$-Menge mindestens geringfügig überschüssigen Mengen eines Protonendonators (organische Säuren oder Mineralsäuren oder Stoffe, die in einem polymeren unlöslichen Grundgerüst bewegliche acide Wasserstoffatome (z.B. in sauren Ionenaustauschern)) unter $CO_2$-Abspaltung in das freie Amin bzw. in ein Aminsalz aus Amin und überschüssigem Protonendonator besitzen überführt, worauf

3.

das Amin isoliert wird, indem das freie Amin fest oder als Flüssigkeit, gegebenenfalls durch Extraktion, erhalten wird oder aus dem Aminsalz mittels Neutralisation durch basische Verbindungen (z.B. Alkali-/Erdalkali-Hydroxide) freigesetzt wird und wie oben angegeben das Amin isoliert wird.

Bei der Herstellung der NCO-Prepolymeren aus Polyisocyanaten und NCO-aktive H-Atome enthaltenden Verbindungen bleiben oftmals geringe Anteile an monomerem

Isocyanat im Präpolymer. Es hat sich gezeigt, daß im erfindungsgemäßen Verfahren die entsprechenden niedermolekularen Polyamine weitgehend oder vollständig aus dem erfindungsgemäßen Polyamin entfernt werden können. Dies geschieht bei der Zersetzung des Carbamats durch organische Säure oder Mineralsäuren dadurch, daß die niedermolekularen, aus monomerem Isocyanat entstammenden Amine in Wasser z.T. löslich sind und mit diesem vom Verfahrensprodukt abgetrennt werden können. Verwendet man als Protonendonatoren Stoffe, die in einem polymeren Grundgerüst bewegliche acide H-Atome, also z.B. Ionenaustauscherharze in saurer Form, so hat sich überraschenderweise gezeigt, daß in dieser Variante des erfindungsgemäßen Verfahrens nur die entsprechenden niedermolekularen Polyamine mit dem Ionenaustauscherharz Assoziate ergeben und mit diesen aus dem Reaktionsgemisch entfernt werden. Überraschenderweise geschieht das jedoch nicht mit den höhermolekularen Polyaminen, so daß nach dem dieser Variante des erfindungsgemäßen Verfahrens höhermolekulare Polyamine direkt sowie mit verbesserter Molekular-Einheitlichkeit zugänglich sind.

Gegenstand der vorliegenden Erfindung ist ferner die Verwendung der nach dem erfindungsgemäßen Verfahren zugänglichen schwefelhaltigen, primären Polyamine mit Urethan- und/oder Harnstoff- und/oder Biuret- und/oder Isocyanuratgruppen zur Herstellung von gegebenenfalls zellförmigen Polyurethankunststoffen durch Umsetzung von

(A) Polyisocyanaten mit
(B) Polyaminen, sowie gegebenenfalls
(C) weiteren niedermolekularen und/oder höhermolekularen Verbindungen mit gegenüber Isocyanaten reaktiven Gruppen, gegebenenfalls
(D) in Anwesenheit an sich bekannter Hilfs- und Zusatzstoffe,

dadurch gekennzeichnet, daß als Komponente (B) die nach dem erfindungsgemäßen Verfahren hergestellten Polyamine der allgemeinen Formel

$$[H_2N-A]_n \cdot B$$

worin
A, B und n wie oben beschrieben sind,

eingesetzt werden.

Die erfindungsgemäßen Verfahrensprodukte stellen bei Raumtemperatur flüssige bis plastische, in organischen Lösungsmitteln wie auch in NCO-reaktive-Gruppen besitzenden Verbindungen im allgemeinen gut lösliche Verbindungen dar. Sie fallen als farblose bis hellgelbe Substanzen an, die sich bei Zutritt von Luftsauerstoff im Falle des Vorliegens aromatischer Aminogruppen rasch braun verfärben.

Oxidationsbeständiger sind die aliphatische Aminogruppen aufweisenden Verfahrensprodukte. Der Gehalt an primären Aminogruppen in den Verfahrensprodukten

beträgt ca. 0,17 bis ca. 7 %, bevorzugt ca. 1,9 bis ca. 6 %.

Die im erfindungsgemäßen Verfahren eingesetzten NCO-Prepolymere der allgemeinen Formel

$$[OCN-A]_n \cdot B$$

worin

A, B und n wie voranstehend definiert sind,

werden in an sich bekannter Weise, z.B. durch Umsetzung von Wasser und/oder hoch- und/oder niedermolekularen, Hydroxy- und/oder Amino- und/oder Thiolgruppen enthaltenden Verbindungen (Molekulargewicht: 60 bis ca. 10 000) mit einem Überschuß an Polyisocyanat hergestellt. Als Polyisocyanat im erfindungsgemäßen Verfahren werden Polyisocyanate der allgemeinen Formel

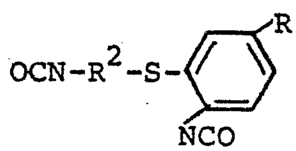

wobei

R Wasserstoff, eine Alkylgruppe mit 1 bis 4 Kohlenstoffatomen oder eine Alkylthiogruppe mit 1 bis 4 Kohlenstoffatomen bedeutet und wobei

$R^2$ für einen, gegebenenfalls durch Alkylgruppen mit vorzugsweise 1 bis 4 Kohlenstoffatomen oder durch eine Alkylthiogruppe mit vorzugsweise 1 bis 4 Kohlenstoffatomen substituierten Phenylrest oder einen linearen oder verzweigten aliphatischen

Kohlenwasserstoffrest mit 2 bis 12 Kohlenstoffatomen steht, wobei zwischen dem Stickstoffatom und dem Schwefelatom mindestens 2 Kohlenstoffatome angeordnet sind,

verwendet. Solche Diisocyanate werden in der DE-A 2 922 966 ausführlich beschrieben. In der gleichen Veröffentlichung sind auch die Harnstoffgruppen aufweisenden, isocyanatgruppenhaltigen Verfahrensprodukte, in denen B für einen Harnstoffrest $-NH-\overset{O}{\overset{\parallel}{C}}-NH-$ steht, beschrieben.

Dabei entstehen, wenn araliphatische, Schwefel enthaltende Diisocyanatoverbindungen verwendet werden, Harnstoffdiisocyanate, deren verbliebene NCO-Gruppe aliphatischen Charakter haben. Erfolgt die Synthese dieser Harnstoffisocyanate durch Reaktion mit Wasser oder Wasserspendern, so entstehen in geringem Ausmaß auch oligomere Harnstoffdiisocyanate.

Beispiele für die erfindungsgemäß einsetzbaren Harnstoffdiisocyanate sind:

1,3-Bis-[2-(4'-isocyanato-3'-ethyl-diphenylsulfid]-harnstoff, 1,3-Bis-[2-(4'-isocyanato-3'-ethylthio-diphenylsulfid)]-harnstoff, 1,3-Bis-[2-(4'-isocyanato-5-ethyl-diphenylsulfid)]-harnstoff, 1,3-Bis-[2-(4'-isocyanato-5-isopropylthio-diphenylsulfid)], 1,3-Bis-[2-(2'-isocyanatoethylthio)-phenyl]-harnstoff, 1,3-Bis-[2-(6'-isocyanatohexylthio)-phenyl]-harnstoff,

1,3-Bis-[2-(2'-isocyanatoethylthio)-4-ethyl-phenyl]-harnstoff, 1,3-Bis-[2-(6'-isocyanatohexylthio)-4-isopropylthio-phenyl]-harnstoff.

Nach an sich bekannten Methoden werden auch die biuretgruppen- und isocyanuratgruppenhaltigen Ausgangsprodukte hergestellt. Auch die Biuretgruppen-haltigen Triisocyanate, wie sie im erfindungsgemäßen Verfahren eingesetzt werden, enthalten für gewöhnlich noch kleinere Anteile (etwa < 10 Gew.-%) an höherfunktionellen Biuretpolyisocyanaten, welche 2 oder mehrere Biuretgruppen in abnehmenden Mengen enthalten.

Beispiele für solche Biuretgruppen-haltigen Ausgangsverbindungen für das erfindungsgemäße Verfahren sind Verbindungen, die der Formel

```
       O
       ‖
  D-N-C-NH-D
     |
     C=O
     |
     NH
     |
     B
```

wobei

D für den 2-(4'-Isocyanato-3'-ethyl-diphenylsulfid)-, 2-(4'-Isocyanato-3'-ethylthio-diphenylsulfid)-, 2-(4'-Isocyanato-5-ethyl-diphenylsulfid)-, 2-[(2'-Isocyanatoethylthio)-phenyl]-, 2-[(6'-Isocyanatohexylthio)-phenyl]-, 2-[(2-Isocyanatoethylthio)-4-ethylphenyl]-, 2-[(6'-Isocyanatohexylthio)-4-isopropylthio-phenyl]-Rest steht,

entsprechen.

Beispiele für Isocyanuratgruppen-haltige Ausgangsverbindungen für das erfindungsgemäße Verfahren sind Verbindungen, die der Formel

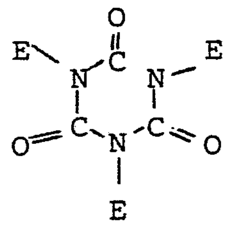

wobei

E für den 2-(4'-Isocyanato-3-ethyl-diphenylsulfid)-, 2-(4'-Isocyanat-3-ethylthio-diphenylsulfid)-, 2-(4'-Isocyanato-5-ethyldiphenylsulfid-, 2-(4'-Isocyanato-3-ethyl-diphenylsulfid)-, 2-(4'-Isocyanato-3-ethylthio-diphenylsulfid)-, 2-(4'-Isocyanato-5-ethyl-diphenylsulfid)-, 2-(4'-Isocyanato-5-isopropylthio-diphenylsulfid)-, 2-[(2'-Isocyanatoethylthio)-phenyl]-, 2-[(6'-Isocyanatohexylthio)-phenyl]-, 2-[(2-Isocyanatoethyl-thio)-4-ethylphenyl]-, 2-[(6'-Isocyanatohexylthio)-4-isopropylthiophenyl]-Rest steht, entsprechen.

Die freie Isocyanatgruppen und Urethangruppen aufweisenden Präpolymeren werden in an sich bekannter Weise durch Umsetzung von Isocyanaten der Formel

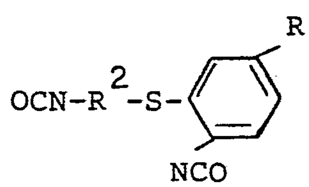

wobei

$R^2$ für einen gegebenenfalls durch Alkylgruppen mit vorzugsweise 1 bis 4 Kohlenstoffatomen oder durch eine

Alkylthiogruppe mit vorzugsweise 1 bis 4 Kohlenstoffatomen substituierten Phenylrest oder einen linearen oder verzweigten aliphatischen Kohlenwasserstoffrest mit 2 bis 12 Kohlenstoffatomen steht, wobei zwischen dem Stickstoffatom und dem Schwefelatom mindestens 2 Kohlenstoffatome angeordnet sind, mit NCO-aktiven H-Atome enthaltenden Verbindungen erhalten.

Als Isocyanate werden zur Prepolymerisierung beispielsweise eingesetzt:

2,4'-Diisocyanato-3'-ethyl-diphenylsulfid, 2,4'-Diisocyanato-3'-ethylthio-diphenylsulfid, 2,4'-Diisocyanato-5-ethyl-diphenylsulfid, 2,4'-Diisocyanato-5-isopropylthio-diphenylsulfid, 2-(2'-Isocyanatoethylthio)-phenylisocyanat, 2-(6'-Isocyanatohexylthio)-phenylisocyanat, 2-(2'-Isocyanatohexylthio)-4-phenylisocyanat.

Zur Herstellung der freien NCO-Gruppen und Urethangruppen aufweisenden NCO-Voraddukte werden als Ausgangskomponenten $G-(OH)_n$ hydroxylgruppenhaltige, niedermolekulare Polyole mit Molekulargewichten ab 62 bis etwa 399 oder höhermolekulare Verbindungen mit mindestens zwei gegenüber Isocyanaten reaktionsfähigen Wasserstoffatomen von einem Molekulargewicht in der Regel von 400 bis 16 000 eingesetzt. Hierunter versteht man neben Aminogruppen, Thiolgruppen oder Carboxylgruppen aufweisenden Verbindungen vorzugsweise Hydroxylgruppen aufweisende Verbindungen, speziell solche vom Molekulargewicht 400 bis 10 000, vorzugsweise 800 bis 6000, z.B. mindestens zwei, vorzugsweise aber 2 bis 4 Hydroxylgruppen aufweisende Polyether, Polythioether, Polyacetale und Polycarbonate, wie sie für die Herstellung von homogenen und von zellförmigen Polyurethanen an sich bekannt sind:

Die erfindungsgemäß in Frage kommenden, mindestens zwei, in der Regel zwei bis vier, vorzugsweise zwei bis drei, Hydroxylgruppen aufweisenden Polyether sind solche der an sich bekannten Art und werden z.B. durch Polymerisation von Epoxiden wie Ethylenoxid, Propylenoxid, Butylenoxid, Tetrahydrofuran, Styroloxid oder Epichlorhydrin mit sich selbst, z.B. in Gegenwart von Lewis-Katalysatoren wie $BF_3$, oder durch Anlagerung dieser Epoxide, vorzugsweise von Ethylenoxid und Propylenoxid, gegebenenfalls im Gemisch oder nacheinander, an Startkomponenten mit reaktionsfähigen Wasserstoffatomen wie Wasser, Alkohole, Ammoniak oder Amine, z.B. Ethylenglykol, Propylenglykol-(1,3) oder -(1,2), Trimethylolpropan, Glycerin, 4,4'-Dihydroxy-diphenylpropan, Anilin, Ethanolamin oder Ethylendiamin hergestellt. Auch auf Formit oder Formose gestartete Polyether (DE-A 2 639 083 bzw. 2 737 951), kommen erfindungsgemäß in Frage. Vielfach sind solche Polyether bevorzugt, die überwiegend (bis zu 90 Gew.-%, bezogen auf alle vorhandenen OH-Gruppen im Polyether) primäre OH-Gruppen aufweisen. Auch OH-Gruppen aufweisende Polybutadiene sind erfindungsgemäß geeignet.

Unter den Polythioethern seien insbesondere die Kondensationsprodukte von Thiodiglykol mit sich selbst und/ oder mit anderen Glykolen, Formaldehyd angeführt. Je nach den Co-Komponenten handelt es sich bei den Produkten z.B. um Polythiomischether oder Polythioether.

Als Polyacetale kommen z.B. die aus Glykolen, wie Diethylenglykol, Triethylenglykol, 4,4'-Dioxethoxydiphenyldimethylmethan, Hexandiol und Formaldehyd herstellbaren Verbindungen in Frage. Auch durch Polymerisation cyclischer Acetale wie z.B. Trioxan (DE-A 1 694 128) lassen sich erfindungsgemäß geeignete Polyacetale herstellen.

Als Hydroxylgruppen aufweisende Polycarbonate kommen solche der an sich bekannten Art in Betracht, die z.B. durch Umsetzung von Diolen wie Propandiol-(1,3), Butandiol-(1,4) und/oder Hexandiol-(1,6), Di-, Tri- oder Tetraethylenglykol oder Thiodiglykol mit Diarylcarbonaten, z.B. Diphenylcarbonat, oder Phosgen hergestellt werden können (DE-B 1 694 080, 1 915 908 und 2 221 751; DE-A 2 605 024).

Zu den Polyamiden zählen z.B. die aus mehrwertigen gesättigten oder ungesättigten Carbonsäuren bzw. deren Anhydriden und mehrwertigen gesättigten oder ungesättigten Diaminen, Polyaminen und deren Mischungen gewonnenen, vorwiegend linearen Kondensate mit niedrigen Schmelzpunkten.

Auch bereits Urethan- oder Harnstoffgruppen enthaltende Polyhydroxylverbindungen sowie gegebenenfalls modifizierte natürliche Polyole, wie Rizinusöl oder Kohlenhydrate, z.B. Stärke, sind verwendbar. Auch Anlagerungsprodukte von Alkylenoxiden an Phenol-Formaldehyd-Harze oder auch an Harnstoff-Formaldehyd-Harze sind erfindungsgemäß einsetzbar.

Die genannten Polyhydroxylverbindungen können vor ihrer Verwendung im Polyisocyanat-Polyadditionsverfahren noch in der verschiedensten Weise modifiziert werden. Es ist auch möglich, z.B. gemäß DE-A 2 559 372 in die Polyhydroxylverbindungen Amidgruppen oder gemäß DE-A 2 620 487 durch Umsetzung mit polyfunktionellen Cyansäureestern Triazingruppen einzuführen. Durch Umsetzung eines Polyols mit einer weniger als äquivalenten Menge eines Diisocyanatocarbodiimids und anschließende Reaktion der Carbodiimidgruppe mit einem Amin, Amid, Phosphit oder einer Carbonsäure erhält man Guanidin-, Phosphonoformamidin- bzw. Acylharnstoffgruppen aufweisende Polyhydroxylverbindungen (DE-A 2 714 289, 2 714 292 und 2 714 293). Von besonderem Interesse ist es in manchen Fällen, die höhermolekularen Polyhydroxylverbindungen durch Reaktion mit Isatosäureanhydrid

vollständig oder teilweise in die entsprechenden Anthranilsäureester überzuführen, wie es in den DE-A 2 019 432 und 2 619 840 bzw. den US-PS 3 808 250, 3 975 428 und 4 016 143 beschrieben ist. Man erhält auf diese Weise höhermolekulare Verbindungen mit endständigen aromatischen Aminogruppen.

Durch Umsetzung von NCO-Prepolymeren mit Hydroxylgruppen aufweisenden Enaminen, Aldiminen oder Ketiminen und anschließende Hydrolyse erhält man gemäß DE-A 2 546 536 bzw. US-PS 3 865 791 höhermolekulare, endständige Aminogruppen aufweisende Verbindungen. Weitere Herstellungsverfahren für höhermolekulare Verbindungen mit endständigen Aminogruppen oder Hydrazidgruppen werden in der DE-A 1 694 152 (US-PS 3 625 871) beschrieben.

Erfindungsgemäß können gegebenenfalls auch Polyhydroxylverbindungen eingesetzt werden, in welchen hochmolekulare Polyaddukte bzw. Polykondensate oder Polymerisate in feindisperser oder gelöster Form enthalten sind. Derartige Polyhydroxylverbindungen werden z.B. erhalten, wenn man Polyadditionsreaktionen (z.B. Umsetzungen zwischen Polyisocyanaten und aminofunktionellen Verbindungen) bzw. Polykondensationsreaktionen (z.B. zwischen Formaldehyd und Phenolen und/oder Aminen) in situ in den obengenannten, Hydroxylgruppen aufweisenden Verbindungen ablaufen läßt. Derartige Verfahren sind beispielsweise in den DE-B 1 168 075 und 1 260 142, sowie den DE-A 2 324 134, 2 423 984, 2 512 385, 2 513 815, 2 550 796, 2 550 797, 2 550 833, 2 550 862, 2 633 293 und 2 639 254 beschrieben. Es

ist aber auch möglich, gemäß US-PS 3 869 413 bzw. DE-A 2 550 860 eine fertige wäßrige Polymerdispersion mit einer Polyhydroxylverbindung zu vermischen und anschließend aus dem Gemisch das Wasser zu entfernen.

Auch durch Vinylpolymerisate modifizierte Polyhydroxylverbindungen, wie sie z.B. durch Polymerisation von Styrol und Acrylnitril in Gegenwart von Polyethern (US-PS 3 383 351, 3 304 273, 3 523 093, 3 110 695; DE-B 1 152 536) oder Polycarbonatpolyolen (DE-PS 1 769 795; US-PS 3 637 909) erhalten werden, sind für das erfindungsgemäße Verfahren geeignet. Bei Verwendung von Polyetherpolyolen, welche gemäß den DE-A 2 442 101, 2 644 922 und 2 646 141 durch Pfropfpolymerisation mit Vinylphosphonsäureestern sowie gegebenenfalls (Meth)acrylnitril, (Meth)acrylamid oder OH-funktionellen (Meth)acrylsäureestern modifiziert wurden, erhält man Kunststoffe von besonderer Flammwidrigkeit. Polyhydroxylverbindungen, in welche durch radikalische Pfropfpolymerisation mittels ungesättigter Carbonsäuren sowie gegebenenfalls weiterer olefinisch ungesättigter Monomerer Carboxylgruppen eingeführt wurden (DE-A 2 714 291, 2 739 620 und 2 654 746) können mit besonderem Vorteil in Kombination mit mineralischen Füllstoffen eingesetzt werden.

Bei der Verwendung von modifizierten Polyhydroxylverbindungen der obengenannten Art als Ausgangskomponente im Polyisocyanat-Polyadditionsverfahren entstehen in vielen Fällen Polyurethankunststoffe mit wesentlich verbesserten mechanischen Eigenschaften.

Vertreter der genannten erfindungsgemäß zu verwendenden Verbindungen sind z.B. in High Polymers, Vol. XVI, "Polyurethanes, Chemistry and Technology", verfaßt von Saunders-Frisch, Interscience Publishers, New York, London, Band I, 1962, Seiten 32-42 und Seiten 44-54 und Band II, 1964, Seiten 5-6 und 198-199, sowie im Kunststoff-Handbuch, Band VII, Vieweg-Höchtlen, Carl-Hanser-Verlag, München, 1966, z.B. auf den Seiten 45-71, beschrieben. Selbstverständlich können Mischungen der obengenannten Verbindungen mit mindestens zwei gegenüber Isocyanaten reaktionsfähigen Wasserstoffatomen mit einem Molekulargewicht von 400 bis 10 000, z.B. Mischungen von Polyethern und Polythioethern, eingesetzt werden.

Von besonderem Vorteil ist es dabei in manchen Fällen, niedrigschmelzende und hochschmelzende Polyhydroxylverbindungen miteinander zu kombinieren (DE-A 2 706 297).

Gegebenenfalls als Ausgangskomponenten Verbindungen mit mindestens zwei gegenüber Isocyanaten reaktionsfähigen Wasserstoffatomen und einem Molekulargewicht von 18, bzw. von 32 bis 399. Auch in diesem Fall versteht man hierunter Wasser, sowie Hydroxylgruppen und/oder Aminogruppen und/oder Thiolgruppen und/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 oder mehrere, vorzugsweise 2 bis 4,

gegenüber Isocyanaten reaktionsfähige Wasserstoffatome auf.

Auch in diesem Fall können Mischungen von verschiedenen Verbindungen mit mindestens zwei gegenüber Isocyanaten reaktionsfähigen Wasserstoffatomen mit einem Molekulargewicht von 32 bis 399 verwendet werden.

Als Beispiele für derartige Verbindungen seien genannt:

Ethylenglykol, Propylenglykol-(1,2) und -(1,3), Butylenglykol-(1,4) und -(2,3), Pentandiol-(1,5), Hexandiol-(1,6), Octandiol-(1,8), Neopentylglykol, 1,4-Bishydroxymethyl-cyclohexan, 2-Methyl-1,3-propandiol, Dibrombutendiol, Glyzerin, Trimethylolpropan, Hexantriol-(1,2,6), Trimethylolethan, Pentaerythrit, Chinit, Mannit und Sorbit, Dianhydromannit, Dianhydrosorbit, Ricinusöl, Di-, Tri- und Tetraethylenglykol, höhere Polyethylenglykole mit einem Molekulargewicht bis 400, Di-, Tri-, Tetra- und höhere Poly-Propylenglykole mit einem Molekulargewicht bis 399, Di-, Tri-, Tetrabutylenglykol und höhere Polybutylenglykole mit einem Molekulargewicht bis 399, 4,4'-Dihydroxy-diphenylpropan, Dihydroxymethylhydrochinon, Ethanolamin, Diethanolamin, N-Methyldiethanolamin, Triethanolamin und 3-Aminopropanol.

Als niedermolekulare Polyole kommen erfindungsgemäß auch die Gemische von Hydroxyaldehyden und Hydroxyketonen ("Formose") bzw. die hieraus durch Reduktion erhaltenen mehrwertigen Alkohole ("Formit") in Frage,

wie sie bei der Selbstkondensation von Formaldehydhydrat in Gegenwart von Metallverbindungen als Katalysator und von zur Endiolbildung befähigten Verbindungen als Co-Katalysator entstehen (DE-A 2 639 084, 2 714 084, 2 714 104, 2 721 186, 2 738 154 und 2 738 512).

Ferner sind geeignete niedermolekulare Polyole vom Molekulargewicht bis 400

Esterdiole der allgemeinen Formeln

$HO-(CH_2)_x-CO-O-(CH_2)_y-OH$ und

$HO-(CH_2)_x-O-CO-R-CO-O-(CH_2)_x-OH$, in denen

R einen Alkylenrest mit 1 bis 10, vorzugsweise 2 bis 6, C-Atomen bzw. einen Cycloalkylen- oder Arylenrest mit 6 bis 10 C-Atomen,

x 2 bis 6 und

y 3 bis 5

bedeuten, z.B. δ-Hydroxybutyl-ε-hydroxy-capronsäureester, ω-Hydroxyhexyl-γ-hydroxybuttersäureester, Adipinsäure-bis-(ß-hydroxyethyl)ester und Terephthalsäure-bis(ß-hydroxyethyl)ester; Diolurethane der allgemeinen Formel

$HO-(CH_2)_x-O-CO-NH-R'-NH-CO-O-(CH_2)_x-OH$

in der

R' einen Alkylenrest mit 2 bis 15, vorzugsweise 2 bis 6, C-Atomen oder einen Cycloalkylen- oder Arylenrest mit 6 bis 15 C-Atomen und

x eine Zahl zwischen 2 und 6

darstellen, z.B. 1,6-Hexamethylen-bis-(ß-hydroxyethylurethan) oder 4,4'-Diphenylmethan-bis-(δ-hydroxybutylurethan);

sowie Diolharnstoffe der allgemeinen Formel

$$HO-(CH_2)_x-\underset{R'''}{N}-CO-NH-R''-NH-CO-\underset{R'''}{N}-(CH_2)_x-OH$$

in der

R" einen Alkylenrest mit 2 bis 15, vorzugsweise 2 bis 9, C-Atomen oder einen Cycloalkylen- oder Arylenrest mit 6 bis 15 C-Atomen,

R'" Wasserstoff oder eine Methylgruppe und

x die Zahlen 2 oder 3

bedeuten,, z.B. 4,4'-Diphenylmethan-bis-(ß-hydroxyethylharnstoff). oder die Verbindung

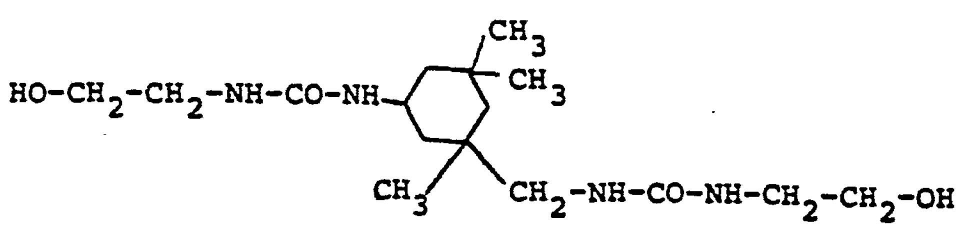

Für manche Zwecke ist es vorteilhaft, Polyole einzusetzen, welche Sulfonat- und/oder Phosphonatgruppen enthalten (DE-A 2 719 372), vorzugsweise das Addukt von Bisulfit an Butandiol-1,4 bzw. dessen Alkoxylierungsprodukte.

Die freie Isocyanatgruppen aufweisenden NCO-Prepolymeren werden in an sich bekannter Weise durch Umsetzung der Reaktionspartner in der Schmelze oder in Lösung hergestellt. Das Äquivalentverhältnis von NCO-Gruppen zu aktiven Wasserstoffatomen (bevorzugt OH-Gruppen) ist in jedem Fall größer als 1, soll aber in der Regel nicht größer als 10 sein. Selbstverständlich ist es möglich, einen noch größeren Überschuß an Polyisocyanat zu verwenden. Das ist aber unnötig, da man das gleiche Ergebnis erreicht, indem man zum NCO-Voraddukt (NCO-Prepolymer) nachträglich monomeres Polyisocyanat hinzufügt. Die Voraddukte haben je nach den gewählten Ausgangskomponenten im allgemeinen ölige bis wachsartige Konsistenz. Beträgt das NCO/OH-Verhältnis mehr als 2, so erhält man im wesentlichen nicht verlängerte NCO-Voraddukte, während NCO/OH-Verhältnisse von unter 2 eine Erhöhung des mittleren Molekulargewichts der NCO-Voraddukte zur Folge haben. Es ist, wie schon erläutert, auch möglich, niedermolekulare Polyole als Kettenverlängerungsmittel bei der Herstellung der NCO-Prepolymeren anteilmäßig neben höhermolekularen Ausgangsverbindungen mitzuverwenden; in diesem Fall erhält man ebenfalls höhermolekulare Voraddukte.

Für das erfindungsgemäße Verfahren werden NCO-Prepolymere bevorzugt, die aus höhermolekularen Polyetherglykolen, gegebenenfalls unter Mitverwendung von Kettenverlängerern der oben beschriebenen Art, und aliphatischen und/oder aromatischen oder araliphatischen, schwefelhaltigen Isocyanaten, wie sie oben beschrieben wurden, im Äquivalentverhältnis von 1:1,5 bis 1:2,8, erhalten worden sind.

Im erfindungsgemäßen Verfahren wird das NCO-Prepolymer zunächst in der Regel in einem mit Wasser mischbaren, inerten Lösungsmittel gelöst. Geeignete Solventien sind z.B. Dimethoxyethan (DME), Tetrahydrofuran oder Dioxan. Bevorzugt ist Dioxan. Dabei können auf 100 Teile Lösungsmittel z.B. 1 bis 400 Teile des NCO-Prepolymeren verwendet werden. Das Prepolymer wird zweckmäßig unter Rühren langsam (vorzugsweise binnen 30 bis 120 Minuten) mit einer auf ca. 0 bis 40°C temperierten Lösung der erfindungsgemäßen Basen, vorzugsweise von Alkali- oder Erdalkalihydroxidenin Wasser, vermischt, wobei die (Erd)-Alkalikonzentration vorzugsweise 1 Gew.-Teil Base auf 5 bis 20 Gew.-Teile Wasser beträgt. Gleichfalls geeignet sind quartäre Ammoniumhydroxide (z.B. Tetraalkylammoniumhydroxide). Als "Basen" sind Oxide und/oder Hydroxide der I., II. und III. Hauptgruppe und/oder quartäre Ammoniumhydroxide für das erfindungsgemäße Verfahren einzusetzen (in der Anmeldung hier als Basen bezeichnet).

Beispiele sind Alkali-, Erdalkalihydroxide, Alkalialuminate, Erdalkalioxide wie Calciumoxid oder Tetraalkylammoniumhydroxide. Bevorzugt sind Hydroxidionen enthaltende Basen wie Alkalihydroxide, Calciumhydroxid oder Tetraalkylammoniumhydroxide wie Tetramethylammoniumhydroxid, wobei diese Basen eine hinreichende Löslichkeit in Wassers aufweisen, um Lösungen zu ergeben. Als Hydroxidion besitzende Basen werden Alkalihydroxide bevorzugt, besonders Natrium- und Kaliumhydroxid.

Wird ohne Solvens gearbeitet, so wird das möglichst niedrigviskose (bevorzugt bis ca. 5000 mPas), gegebenenfalls vorher erwärmte (z.B. 30 bis 90°C) NCO-Prepolymer bei hoher Rührgeschwindigkeit möglichst fein verteilt zugegeben (z.B. Einspritzung durch eine Düse) und zur Rührerleichterung die vorgelegte Wassermenge gegebenenfalls erhöht (z.B. um den Faktor 1.1-100).

Die (Erd)Alkalihydroxidmenge wird so bemessen, daß nach vollständiger Reaktion zumindest noch eine geringe Menge freier Base verbleibt, ein NCO/$OH^{\ominus}$-Ionen-Verhältnis von 1:1,01 bis 1:1,8 und die Verwendung von Alkalihydroxiden wird dabei bevorzugt. Die Restbasenkonzentration darf nicht zu hoch sein, da sonst nach der Bildung des Carbamats im Präpolymer enthaltende Urethangruppierungen gleichfalls hydrolysiert werden können. Um die Homogenität der Lösungen zu verbessern, wird vorzugsweise ein handelsüblicher Emulgator in Mengen von 0,05 bis 1 Gew.-Teilen, bevorzugt ca. 0,1 bis 0,5 Gew.-Teile (bezogen auf 100 Teile Reaktionsgemisch) zugesetzt. Intensives Rühren ist bei der Vermischung der NCO-Komponente mit der Hydroxidkomponente zu empfehlen, um lokale Konzentrationsungleichgewichte zu vermeiden. Nach der Beendigung der Zugabe des NCO-Prepolymeren wird vorzugsweise noch ca. 15 bis 180 Minuten bei 10 bis 25°C weitergerührt.

Die acidolytische Zersetzung der Carbamate wird in 2 Verfahrensvarianten vorgenommen. In der Verfahrensvariante A wird als Protonendonator eine polymere Säure (saures Ionenaustauscherharz), in der Verfahrensvariante B eine starke organische Säure oder Mineralsäure verwendet.

Verfahrensvariante A

Als saure Ionenaustauscher im erfindungsgemäßen Verfahren sind alle Stoffe geeignet, die in einem polymeren unlöslichen Grundgerüst bewegliche acide Wasserstoffatome besitzen. Besonders geeignet als polymere Säuren sind im erfindungsgemäßen Verfahren Ionenaustauscherharze, die als polymere Basis ein Styrol/Divinylbenzol-Skelett besitzen, an das Sulfonsäuregruppen als saure Funktionen gebunden sind.

Im zweiten Schritt wird die Carbamatlösung bzw. Carbamatsuspension mit dem Ionenaustauscher vereinigt. Für das erfindungsgemäße Verfahren ist es unwesentlich, ob zur vorgelegten Carbamatsuspension bzw. Carbamatlösung das saure Ionenaustauscherharz zugegeben (Verfahren 1) oder ob umgekehrt vorgegangen wird (Verfahren 2). Ionenaustauscherharz und Carbamatkomponente werden in dem Maße miteinander vereinigt, wie es die Heftigkeit der Gasentwicklung und die Dimensionierung der Apparatur zuläßt (10 bis 300 Minuten).

Diese Gasentwicklung tritt jedoch erst nach der Zugabe von ca. 1/4 der Gesamtmenge an Ionenaustauscherharz ein.

Bei der Vereinigung beider Komponenten tritt keine oder nur geringe Temperaturerhöhung auf; ein gegebenenfalls durch externes Heizen einzustellender Temperaturbereich von 10 bis 70°C erwies sich als günstig.

Es wird solange Ionenaustauscher zugesetzt, bis keine Gasentwicklung zu erkennen ist. Kurzzeitiges Erwärmen auf 60 bis 100°C treibt gelöstes Kohlendioxid aus. Pro Alkaliäquivalent werden mindestens geringfügig überschüssige Mengen, vorzugsweise aber 1,01 bis 1,8 Äquivalente von dem sauren Ionenaustauscherharz entstammende Wasserstoffionen eingesetzt.

Das umgekehrte Verfahren - Ionenaustauscher vorgelegt - ist v.a. dann zu bevorzugen, wenn die Reaktionsdaten bekannt sind (Verfahren 2), sowie bei kontinuierlicher Arbeitsweise. Am Endpunkt der Reaktion ist das Reaktionsgemisch basisch, wie es dem Gehalt an freiem Amin und dessen Basenstärke entspricht.

Die einfache Aufarbeitung gestaltet sich wie folgt:

Die flüssige, das aminische, flüssige oder gelöste Verfahrensprodukt enthaltende Phase und der beladene Ionenaustauscher (Festphase) werden durch Filtration voneinander getrennt. Bevorzugt wird der beladene Ionenaustauscher mit einem geeigneten Lösungsmittel (z.B. Methanol, Ethanol, Dioxan) gewaschen und mit dem 1. Filtrat vereinigt.

Nach dem Abdestillieren des Solvens (z.B.17 mbar/100°C und 0,1 bis 1,3 mbar/100°C) hinterbleiben die Verfahrensprodukte als farblose bis hellgelbe Flüssigkeiten oder plastische Feststoffe.

Verfahrensvariante B

Nach Beendigung der Zugabe des NCO-Prepolymeren wird vorzugsweise noch ca. 15 bis 180 Minuten bei ca. 10 bis 25°C weitergerührt. Danach wird konzentrierte Mineralsäure (z.B. Schwefel- oder Phosphorsäure, bevorzugt HCl) oder eine starke organische Säure (Ameisensäure, Essigsäure, Trifluoressigsäure etc.) unter starkem Rühren und Kühlen in dem Maße zugegeben,

wie es die Heftigkeit der $CO_2$-Entwicklung zuläßt (ca. 30 Minuten bis 2 Stunden). Das Säure/Basen-Äquivalentverhältnis beträgt bevorzugt ca. 1,2:1 bis 2,5:1, insbesondere 1,5:1 bis 2,0:1, so daß das Reaktionsprodukt schwach sauer ist (pH bevorzugt $<6$, insbesondere ca. 5,5 bis 1). Auf 100 g eingesetztes NaOH werden somit vorzugsweise 300 bis 500 ml konzentrierte (37 %) HCl verwendet. Es wird vorzugsweise noch ca. 10 bis 30 Minuten nachgerührt und die überschüssige Mineralsäure durch Basenzusatz (bevorzugt mit Alkalihydroxid) neutralisiert.

Zur weiteren Aufarbeitung kann z.B. wie folgt vorgegangen werden, insbesondere, wenn Dioxan als bevorzugtes Lösungsmittel für das NCO-Prepolymer verwendet wurde:

1) Bilden sich (bei kurzkettigen und hydrophoben Polyaminen) zwei Phasen aus, nämlich eine Diamin/Dioxan- und eine Dioxan/Salz/Wasser-Phase, so wird die wäßrige Phase mittels eines Scheidetrichters abgetrennt und verworfen oder gegebenenfalls mit Dichlormethan (250 ml $CH_2Cl_2$ auf 1 l wäßrige Lösung) ausgeschüttelt. Die, gegebenenfalls mit dem $CH_2Cl_2$-Extrakt aus der wäßrigen Lösung vereinigte Aminphase, wird auf Chlor geprüft. Sind lediglich Mengen $<1$ % $Cl^{\ominus}$ (oder eines anderen Anions) enthalten, wird bei 10 bis 20 Torr das Lösungsmittel abdestilliert.

Sollte nach der $Cl^{\ominus}$-Bestimmung noch Hydrochlorid (bzw. ein anderes Säuresalz) enthalten sein, so wird die dioxanische Lösung (auch $CH_3OH$ etc.) über einer berechneten Menge basischen Ionenaustauschers (z.B. ® Lewatit WP 500) vom Salz befreit. So werden Produkte mit einem Salzgehalt < 1 % erhalten. Es ist auch möglich, bei 10 bis 30°C, bevorzugt bei Raumtemperatur, durch Rühren (2 bis 8 h) einer Suspension dieses Ionenaustauschers in der Aminlösung den gleichen Effekt zu erzielen. Es kann auch so vorgegangen werden, daß das Dioxan abdestilliert, mit Dichlormethan aufgefüllt und die so erhaltene Polyamin-Lösung durch mehrmaliges Ausschütteln mit Wasser (ca. 1 l $H_2O$ auf 5 l $CH_2Cl_2$-Lösung) salzfrei gemacht wird.

2) Bilden sich keine zwei Phasen, wird wie folgt vorgegangen:

Auf 1 l des Reaktionsgemisches werden 500 ml Dichlormethan gegeben und in einem Scheidetrichter gut geschüttelt. Nach dem Absetzenlassen der beiden Phasen wird die außer dem Polyamin noch Dioxan enthaltende $CH_2Cl_2$-Lösung abgetrennt. Dieser Vorgang wird 2 bis 5 mal wiederholt und anschließend die $CH_2Cl_2$-Phasen vereinigt. Durch Abdestillation aller flüchtigen Anteile wird ein lösungsmittelfreies Polyamin erhalten, das, sollte es noch $Cl^{\ominus}$ enthalten, weiter wie oben aufgearbeitet wird.

Es kann auch so vorgegangen werden, daß die Salzanteile mit Wasser aus dem dioxanischen Reaktionsgemisch extrahiert werden (Scheidetrichter: 1 l Wasser auf 1 bis 5 l Reaktionsgemisch).

Bei zu langsamer Separation der beiden Phasen (Vorversuch) wird auf eine Dichlormethan- bzw. Wasserzugabe verzichtet und das gesamte Reaktionsgemisch bei 10 bis 20 Torr/40 bis 60°C Badtemperatur von flüchtigen Bestandteilen befreit. Der so erhaltene, noch Alkalisalze enthaltende, ölige Rückstand wird in org. Solventien wie z.B. $CH_2Cl_2$, Methanol, Ethanol, Dioxan etc. aufgenommen und durch Filtration (Papierfilter, Drucknutsche) von ungelöstem Salz abgetrennt. Sollte das Polyamin noch Salz enthalten, wird wie oben vorgegangen.

Bei auf bei Raumtemperatur harzartigen NCO-Prepolymeren beruhenden Polyaminen kann auch so vorgegangen werden, daß der Aminpolyether mit gesättigter Kochsalzlösung aus der Reaktionsmischung ausgefällt wird; das Polyamin wird durch Dekantierung isoliert, im Vakuumtrockenschrank (60 bis 100°C, 2 bis 16 h, 16 bis 18 Torr) getrocknet, wieder in organischen Solventien (z.B. $CH_2Cl_2$, Dioxan, THF, DME), gelöst, von unlöslichen Salzanteilen abfiltriert und das Lösungsmittel abdestilliert.

Alle so erhaltenen Polyamine werden bei 0,01 bis 0,1 Torr/60°C von Spuren flüchtiger Bestandteile befreit.

Verfahrensvariante A wird bevorzugt zur Herstellung aromatischer, Schwefel enthaltender primärer Amine, Verfahrensvariante B bevorzugt zur Herstellung aliphatischer, primärer, Schwefel enthaltender Polyamine eingesetzt.

Die bei Verfahrensvariante A anfallenen beladenen Ionenaustauscherharze lassen sich nach bekannten Methoden regulieren und erneut problemlos einsetzen.

Die erfindungsgemäß erhaltenen Polyamine werden wegen ihres niedrigen Dampfdrucks vorzugsweise als Reaktionspartner für Polyisocyanate bei der Herstellung von gegebenenfalls zelligen Polyurethankunststoffen eingesetzt, wobei sie gegebenenfalls auch mit anderen niedermolekularen (Molekulargewicht: 32 bis 399) und/oder höhermolekularen (Molekulargewicht: 400 bis ca. 16 000, vorzugsweise 400 bis 10 000) Verbindungen mit gegenüber Isocyanaten reaktiven Gruppen kombiniert werden können. Geeignete Ausgangskomponenten für die Herstellung von Polyurethankunststoffen werden oben im Zusammenhang mit der NCO-Prepolymerherstellung bzw. auch in DE-A 2 302 564, DE-A 2 432 764 (US-PS 3 963 679) sowie in den DE-A 2 639 083, 2 512 385, 2 513 815, 2 550 796, 2 550 797, 2 550 833, 2 550 860 und 2 550 862 genannt. Dort finden sich auch Hinweise auf bei der Polyurethanherstellung gegebenenfalls mitzuverwendende Hilfs- und Zusatzstoffe. Die Herstellung von Polyurethanharnstoffen mittels der erfindungsgemäß hergestellten Polyamine ist ebenfalls Gegenstand der vorliegenden Erfindung.

Weitere Verwendungszwecke der erfindungsgemäß hergestellten Polyamine sind z.B. Kupplungskomponenten für Diazafarbstoffe, Härter für Epoxid- und Phenolharze sowie alle anderen an sich bekannten Reaktionen von Aminen wie Amid- oder Imidbildung etc.

Die vorliegenden Beispiele dienen der Erläuterung des erfindungsgemäßen Verfahrens. Wenn nicht anders vermerkt, sind Mengenangaben als Gewichtsteile bzw. als Gewichtsprozente zu verstehen.

I. Herstellungsbeispiele

Beispiel 1

1.1 Herstellung des Carbamats

Eine Lösung aus 941 g eines durch Umsetzung von 2,4'-Diisocyanatodiphenylsulfid mit Wasser in Aceton hergestellten, einen NCO-Wert von 15 % aufweisenden Harnstoffs in 640 ml wasserfreiem Dioxan wird auf 50°C erwärmt und in 45 Minuten zu einer Lösung von 224 g (4 Mol) KOH und 1,6 g Mersolat® H in 640 ml Wasser getropft. Das molare KOH/NCO-Verhältnis beträgt 1,2:1. Im Reaktionsgefäß wird während des Zutropfens und des 30-minütigen Nachrührens eine Innentemperatur von 18 bis 22°C aufrechterhalten.

1.2 Herstellung des Amins

Die bei der Herstellung des Carbamats anfallende feinteilige Dispersion wird in zwei Hälften geteilt.

1.2.1 mit Ionenaustauscher

1200 g des obigen Reaktionsgemisches werden bei 20°C in 30 Minuten portionsweise mit 1,6 l eines sauren Ionenaustauschers (Lewatit® SPC 118-BAYER) versetzt. Dabei entwickeln sich 34 l $CO_2$ (92 % der Theorie, berechnet auf Mono-Harnstoff). Der Ionenaustauscher wird nach 60 Minuten Nachrühren bei 45°C abgesaugt und in 400 ml Dimethylformamid (20°C) gegeben. Das

Ionenaustauscherharz wird abgesaugt und aus dem Filtrat das Lösungsmittel abdestilliert. Es resultieren 376 g eines harzig-zähflüssigen, braungelben Produkts. Die Wasser-Dioxan-Phase enthält kein Produkt.

Produktdaten:

NH-Zahl: 207 (th. für Monoharnstoff: 244)
Säure-Zahl: 0,23
Molmasse (dampfdruckosmometrisch in Dimethylformamid): 513 (th. für Monoharnstoff: 458).

Die Daten zeigen, daß teilweise über Harnstoffgruppen verlängertes Diamin vorliegt.

Lewatit ® SPC 118 (Bayer AG) ist ein stark saurer Kationenaustauscher mit $-SO_3H$-Gruppen in einer Styrol/Divinylbenzol-Matrix (DVB-Gehalt 18 %). Der makroporösgroßporige Austauscher mit einem Kornspektrum von 0,3 bis 1,5 mm hat eine Totalkapazität von 1,4 bis 1,5 mval pro ml.

Es wurden in gleichartiger Durchführungsform folgende Ionenaustauscherharze im Verfahren des Beispiels eingesetzt, wobei das Polyamin in vergleichbarer Ausbeute und Reinheit erhalten wurde: Lewatit SPC 108, SC 108, SC 104 und CNP 80.

Angaben zu diesen Ionenaustauschern finden sich in den Schriften der Bayer AG, z.B. der unter der Nr.

OC/I 20 333 (Ausgabe vom 1.8.1979) erschienenen Schrift.

1.2.2 mit Mineralsäure

Zu 1200 g des obigen Reaktionsgewichts werden bei 15 bis 20°C innerhalb 40 Minuten 338 ml konz. HCl zugegeben. Dabei konnten 35,4 l (96 % der Theorie, bezogen auf Monoharnstoff) gemessen werden. Nach Beendigung der Gasentwicklung wird mit einer Lösung von 29,1 g KOH in 150 ml Wasser neutralisiert und der gesamte Reaktionsansatz einer Vakuumdestillation unterworfen. Dabei wird zuerst im Wasserstrahlpumpenvakuum destilliert, dann mit 250 ml Toluol versetzt, abfiltriert, abermals im Wasserstrahlpumpenvakuum destilliert und zum Schluß 2 h/100°C bei 0,1 Torr letzte Spuren flüchtiger Begleitstoffe entfernt. Es werden 398 g eines dunkelbraunen zähen Harzes erhalten. Entsprechende Harnstoffamine auf Basis von 2,4-Toluylendiisocyanat und 2,4'- oder 4,4'-Diisocyanatodiphenylmethan stellen hochschmelzende, kristalline Verbindungen dar.

Produktdaten:

NH-Zahl: 194 (th. für Monoharnstoff: 244)
Säurezahl: 1,2
Molmasse (dampfdruckosmometrisch in Dimethylformamid): 570 (th. für Monoharnstoff: 458).

Die Daten zeigen, daß teilweise über Harnstoffgruppen verlängertes Diamin vorliegt.

Beispiel 2

2.1 Herstellung des Carbamats

400 g eines auf 60°C erwärmten NCO-Prepolymers mit einem NCO-Gehalt von 3,24 %, das hergestellt worden ist aus einem auf Propylenglykol gestarteten Polypropylenglykolether der Molmasse 2000 und 2,4'-Diisocyanatodiphenylsulfid im NCO:OH-Verhältnis von 2:1, werden in 20 Minuten zu einer vorgelegten Lösung aus 26 g (0,463 Mol) KOH und 0,4 g Mersolat® H in 400 ml Wasser gegeben, wobei eine Innentemperatur von 18 bis 20°C aufrechterhalten wird. Es wird noch weitere 20 Minuten gerührt und anschließend mit 1,3 l Methanol versetzt.

2.2 Herstellung des Amins

In dieses mit Methanol verdünnte Reaktionsgemisch werden 260 ml feuchtes Lewatit® SC 108 zugegeben. Die anfänglich nur zögernde Kohlendioxid-Entwicklung vervollständigt sich beim Erwärmen auf 55°C. Es lassen sich 6 l $CO_2$ (87 % der Theorie), bezogen auf NCO-Wert des NCO-Prepolymers, messen. Das Lewatit (beladene Form) wird durch Filtration abgetrennt, das Filtrat durch Destillation im Wasserstrahl- und später im Ölpumpenvakuum von den Lösungsmitteln befreit. Es resultieren 330 g (84 %) eines niedrig viskosen, hellgelben Öles.

Produktdaten:

NH-Zahl: 39,9 (th. 45)
Säurezahl: 1,6
Molmasse: 2895 (th. 2807)
Wassergehalt (Karl Fischer): 0,64 %
Viskosität $\eta^{20}$: 33 000 mPas

Beispiel 3

3.1 Herstellung des Carbamats

500 g eines aus 2,4'-Diisocyanato-diphenylsulfid und einem Polypropylenglykolether des Molekulargewichts 1000 im NCO:OH-Verhältnis von 2:1 hergestellten und einen NCO-Wert von 5,9 % aufweisenden NCO-Prepolymers werden auf 65°C erwärmt und innerhalb 60 Minuten in eine vorgelegte Lösung von 28,1 g (1,05 Mol) Natriumhydroxid und 0,6 g Mersolat® H in 500 ml Wasser getropft. Dabei wird eine Innentemperatur von 25 bis 30°C nicht überschritten (externe Kühlung). Danach wird 90 Minuten bei Raumtemperatur nachgerührt und anschließend mit 600 g n-Propanol verdünnt.

3.2 Herstellung des Amins

Unter Eiskühlung wird zur Reaktionsmischung 60 ml 50 %ige Schwefelsäure gegeben, worauf 15,1 l Kohlendioxid (96 % der Theorie, bezogen auf den NCO-Gehalt des Ausgangsprepolymers) freigesetzt werden. Die überschüssige Schwefelsäure wird mit 6,8 g Natriumhydroxid in 50 ml Wasser neutralisiert. Danach wird der gesamte Reaktionsansatz durch Destillation von den Lösungsmitteln befreit, der Rückstand mit 500 ml Dichlormethan aufgenommen und das Natriumsulfat abgesaugt. Die $Na_2SO_4$-Fällung wird mit 200 ml Dichlormethan gewaschen, Filtrat und Waschphase vereinigt und das Dichlormethan bei 18 mbar/60°C und dann 0,12 mbar/100°C abdestilliert. Es hinterbleibt ein braunes, zähflüssiges Öl (440 g, 91 % der Theorie).

Produktdaten:

NH-Zahl: 60,3, 60,7
Säurezahl: 0,9
Molmasse: 1900
Viskosität $\eta^{20}$: 56 000 mPas
Wassergehalt (Karl Fischer): 0,4 %

Beispiel 4

4.1 Herstellung des Carbamats

500 g eines einen NCO-Wert von 3,24 % aufweisenden NCO-Prepolymers (aus 2-(6'-Isocyanatohexylthio)-phenylisocyanat und einem auf Propylenglykol gestarteten Polypropylenglykolethers der Molmasse 2000 (NCO:OH-Verhältnis 2:1)) werden auf 60°C erwärmt und binnen 30 Minuten zu einer Lösung von 27,6 g Kaliumhydroxid und 0,5 g Mersolat® H in 500 ml Wasser gegeben. Dabei wird unter Rühren eine Innentemperatur von 18 bis 22°C eingehalten. Nach Beendigung der Zugabe des NCO-

Prepolymers wird ohne weitere Kühlung 15 Minuten nachgerührt und anschließend mit 1,4 l Methanol verdünnt.

4.2 Herstellung des Polyamins

Obige Reaktionsmischung wird nun mit 270 ml feuchtem Lewatit SC 108 versetzt. Die bei Raumtemperatur nur sehr langsame Kohlendioxidentwicklung wird durch Erwärmen auf 60°C verstärkt. Nach 2 Stunden haben sich 6,3 l (86 % der Theorie, bezogen auf NCO-Gehalt des Ausgangs-NCO-Prepolymers) entwickelt. Nach Beendigung der $CO_2$-Entwicklung wird die Reaktionsmischung abgesaugt, das beladene Ionenaustauscherharz mit 250 ml Ethanol gewaschen, Filtrat und Waschwasser vereinigt und die Lösungsmittel im Wasserstrahlpumpenvakuum abdestilliert. Die restlichen flüchtigen Bestandteile werden bei 0,11 mbar/100°C entfernt. Es resultiert ein gelbes Öl in 83,5 % Ausbeute (410 g).

Produktdaten:

NH-Zahl: 37,4, 37,9
Säurezahl: 0,1
Molmasse: 3000
Viskosität $\eta^{20}$: 14 000 mPas
Wassergehalt (Karl Fischer): 0,05 %

<u>Beispiel 5</u>

5.1 Herstellung des Carbamats

0071138



400 g eines aus 2-(6'-Isocyanatohexylthio)-phenylisocyanat und einem auf Propylenglykol gestarteten Polypropylen/Polyethylenglykolmischpolyether des Molekulargewichts 4000 im NCO/OH-Verhältnis von 2:1 hergestellten und einen NCO-Wert von 2,13 % aufweisenden NCO-Prepolymers werden auf 55°C erwärmt und innerhalb 30 Minuten zu einer Lösung von 17 g Kaliumhydroxid und 0,4 g Mersolat® H in 400 ml Waser gegeben ($OH^-$:NCO-Verhältnis (molar) = 1,5). Während der Zugabe wird eine Innentemperatur von 23 bis 25°C aufrechterhalten. 15 Minuten wird ohne externe Kühlung weitergerührt und dann mit 800 ml i-Propanol versetzt.

5.2 Herstellung des Amins

Zu obiger Reaktionsmischung wird auf einmal 170 ml Lewatit® zugegeben, worauf sofort bei Raumtemperatur kräftige Gasentwicklung auftritt, die durch kurzes Erwärmen auf 50°C vervollständigt wird. Es entweichen 4,5 l Kohlendioxid (100 % der Theorie, bezogen auf den NCO-Gehalt des NCO-Prepolymers). Der beladene Ionenaustauscher wird abgesaugt und mit 360 ml Methanol nachgewaschen. Filtrat und Waschlösung werden vereinigt und das Lösungsmittel bei 17 mbar/80°C abdestilliert. Nachdem bei 0,11 mbar/100°C flüchtige Restmengen entfernt werden, resultieren 350 g (89 % der Theorie) eines gelben Öls.

Produktdaten:

NH-Zahl: 16,8
Säurezahl: 0,2
Molmasse: 6600
Viskosität $\eta^{20}$: 16 000 mPas
Wassergehalt (Karl Fischer): 0,1 %

Beispiel 6

6.1 Herstellung des Carbamats

580 g eines aus 2-(6'-Isocyanatohexylthio)-phenylisocyanats und einem auf Trimethylolpropan gestarteten Polypropylen/Polyethylenglykolmischpolyethers des Molekulargewichts 6000 im NCO:OH-Verhältnis von 2:1 hergestellten und einen NCO-Wert von 1,88 % aufweisenden NCO-Prepolymers werden in 300 ml wasserfreiem Dioxan gelöst und innerhalb 30 Minuten zu einer Lösung von 15,6 g Natriumhydroxid (390 mmol) und 1 g Mersolat® H in 300 ml wasserfreiem Tetrahydrofuran gegeben. Durch Kühlung wird dabei eine Innentemperatur von 16 bis 20°C aufrechterhalten. Nach 20 Minuten Nachrühren ohne externe Kühlung wird mit 500 ml Methanol verdünnt.

6.2 Herstellung des Amins

Obige Reaktionsmischung wird bei Raumtemperatur innerhalb 5 Minuten zu vorgelegten 220 ml feuchtem Lewatit® SC 108 unter Rühren eingetragen. Die stetige Gasentw icklung, die sofort eintritt, wird durch Erwärmen auf 50°C verstärkt. Nach 90 Minuten werden 5,8 l Kohlendioxid (100 % der Theorie, bezogen auf den NCO-Wert des Ausgangs-NCO-Prepolymers) gemessen. Das beladene Ionenaustauscherharz wird abfiltriert, mit 400 ml Ethanol gewaschen, Filtrat und Waschlösung vereinigt, das Lösungsmittel unter vermindertem Druck abdestilliert. Bei 100°C/0,11 mbar schließlich werden Reste flüchtiger Stoffe entfernt. Es resultieren 350 g (89 % der Theorie) eines gelben Öles.

Produktdaten:

NH-Zahl: 19
Säurezahl: 1,3
Molmasse: 8800
Viskosität $\eta^{20}$: 25 000 mPas
Wassergehalt (Karl Fischer): 0,4 %

Beispiel 7

Vergleich der Viskositäten (rotationsviskosimetrisch) verschiedener Aminopolyether mit den erfindungsgemäßen, S-enthaltenden Aminopolyethern:

| | Molmasse | $\eta^{20}$ (mPas) |
|---|---|---|
| Aminopolyether nach Beispiel 2 | 2900 | 33 000 |
| Aminopolyether nach Beispiel 3 | 1900 | 56 000 |
| Aminopolyether nach Beispiel 4 | 3000 | 14 000 |
| Aminopolyether nach Beispiel 5 | 6600 | 16 000 |
| Aminopolyether nach Beispiel 6 | 8800 | 25 000 |

Zum Vergleich:

| | Mol-masse | $\eta^{20}$ (mPas) |
|---|---|---|
| Aminopolyether I | 2700 | 63 000 |
| Aminopolyether II | 2100 | > 100 000 |
| Aminopolyether III | 2400 | 16 000 |
| Aminopolyether IV | 4700 | 16 000 |
| Aminopolyether V | 7000 | 26 000 |

Aminopolyether I:

Hergestellt durch NCO-Prepolymerhydrolyse aus einem aus 4,4'-Diisocyanatodiphenylmethan und einem Polypropylenglykolether des durchschnittlichen Molekulargewichts 2000 hergestellten NCO-Prepolymers.

Aminopolyether II:

Hergestellt durch Prepolymerhydrolyse eines aus 4,4'-Diisocyanatodiphenylmethan und einem Polypropylenglykol des durchschnittlichen Molekulargewichts 1000 hergestellten NCO-Prepolymers.

Aminopolyether III:

Hergestellt durch Prepolymerhydrolyse eines aus 2,4-Diisocyanatotoluol und einem Polypropylenglykolether der durchschnittlichen Molmasse 2000 hergestellten NCO-Prepolymers.

Aminopolyether IV:

Hergestellt durch Prepolymerhydrolyse eines aus 2,4-Diisocyanatotoluol und einem Mischpolypropylenglykolpolyethylenglykolether der durchschnittlichen Molmasse 4000 hergestellten NCO-Prepolymers.

Aminopolyether V:

Hergestellt durch Prepolymerhydrolyse eines aus 2,4-Diisocyanatotoluol und einem auf Trimethylolpropan gestarteten Mischpolypropylenglykolpolyethylenglykolether der durchschnittlichen Molmasse 6000 hergestellten NCO-Prepolymers.

## II. Anwendungsbeispiele

### Beispiel 8

150 g eines erfindungsgemäßen, primäre Aminogruppen aufweisenden Polyethers auf Basis eines aus 2-(6-Isocyanatohexylthio)-phenylisocyanat und einem Polypropylenglykolether des durchschnittlichen Molekulargewichts 2000 hergestellten NCO-Prepolymers, der eine NH-Zahl von 37,6 besitzt und eine Viskosität von $\eta^{20}$ von 1400 mPas aufweist, werden auf 50°C erwärmt und mit 81,7 g eines 50°C warmen, einen NCO-Wert von 6,03 Gew.-% aufweisenden NCO-Prepolymers, das durch Umsetzung eines Polypropylenglykolethers des durchschnittlichen Molekulargewichts 1000 mit einem zu 80 % aus 2,4-Toluylendiisocyanat und 20 % 2,6-Toluylendiisocyanat bestehenden Toluylendiisocyanat-Isomerengemisch erhalten wurde, vermischt und auf eine 100°C heiße Platte gegossen.

Gieß-, Aushärtzeit, sowie mechanische Daten des erhaltenen Elastomers sind in Tabelle 1 aufgeführt.

### Beispiel 9

200 g eines nach dem erfindungsgemäßen Verfahren hergestellten, primäre Aminogruppen aufweisenden Polyethers auf Basis eines aus 2-(6'-Isocyanatohexylthio)-phenylisocyanat und einem linearen Polypropylenglykol/Polyethylenglykolmischblockcopolyether des durchschnittlichen Molekulargewichts 4000, hergestellten NCO-Prepolymers, der eine NH-Zahl von 16,8 und eine

Viskosität $\eta^{20}$ von 25 000 mPas aufweist, werden auf 50°C erwärmt und mit 55 g des NCO-Prepolymers aus Beispiel 7 vermischt und auf eine 110°C heiße Platte gegossen.

Gießzeit, Aushärtezeit sowie mechanische Daten des erhaltenen Elastomers sind in Tabelle 1 aufgeführt.

Tabelle 1

| | Beispiel 8 | Beispiel 9 |
|---|---|---|
| Vermischungsverhältnis Aminopolyether/NCO-Voraddukt | 100:54,5 | 100:30,2 |
| Kennzahl | 110 | 110 |
| Gießzeit | 2 min | 5 min |
| Aushärtezeit | 3 min | 10 min |
| (DIN 53 517) nach 24h/70°C [%] | 112 | 4 |
| Shore Härte A (DIN 53 505) | 26 | 38 |
| Elastizität (DIN 53 512 [%] | 32 | 44 |
| Zugversuch nach DIN 53 504 | | |
| $\sigma_{100}$ [MP] | - | 0,7 |
| $\sigma_{300}$ [MP] | - | - |
| Reißspannung [MP] | 0,33 | 0,85 |
| Reißdehnung [%] | 66 | 127 |

Patentansprüche

1) Primäre, aromatische oder aliphatische Polyamine der allgemeinen Formel

$$[H_2N-A]_n \cdot B$$

worin

n eine ganze Zahl von 2 bis 4 bedeutet,

B für n-wertige Reste der Strukturen

$-NH-\overset{O}{\overset{\|}{C}}-NH-$ (n = 2), 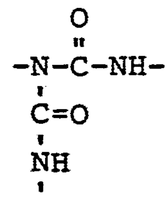 (n = 3),

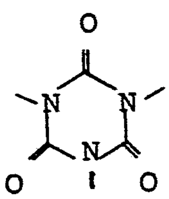 (n = 3), $G(O-\overset{O}{\overset{\|}{C}}-NH-)_n$ (n = 2-4),

G für einen Rest, wie er durch Entfernung von n OH-Gruppen aus n-wertigen Polyhydroxylverbindungen des Molekulargewichtsbereichs 62-10 000 erhalten wird, und

A für einen aromatischen Rest der allgemeinen Formel

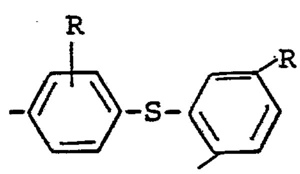

worin

R einen Alkyl- oder Alkylthiorest mit 1 bis 4 Kohlenstoffatomen bedeutet,

oder einen Rest der Formel

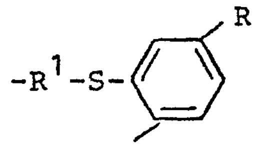

worin

R eine Alkyl- oder Alkylthiogruppe mit 1 bis 4 Kohlenstoffatomen und

$R^1$ einen linearen oder verzweigten aliphatischen Kohlenwasserstoffrest mit 2 bis 12 Kohlenstoffatomen bedeuten,

stehen.

2) Verfahren zur Herstellung Schwefel enthaltender, gut löslicher, niedrig schmelzender oder flüssiger, niedrigviskoser primärer aromatischer oder aliphatischer Polyamine der allgemeinen Formel

$$[H_2N-A]_n \cdot B$$

worin
A,B und n wie oben beschrieben sind,

durch Vermischen von NCO-Voraddukten mit Basen aus der I., II. und III. Hauptgruppe des Periodensystems und/oder quartären Ammoniumhydroxiden in Gegenwart von Wasser unter Bildung des Carbamats, Überführung des Carbamats durch Zusatz einer zur $OH^{\ominus}$-Menge mindestens geringfügig überschüssigen Menge eines Protonendonators unter $CO_2$-Abspaltung in das freie Amin bzw. sein Salz und gegebenenfalls Freisetzung des Amins durch Alkali, dadurch gekennzeichnet, daß man ein Urethan- und/oder Harnstoff- und/oder Biuret- und/oder Isocyanuratgruppen aufweisendes NCO-Voraddukt der allgemeinen Formel

$$[OCN-A]_n \cdot B$$

worin

n eine ganze Zahl von 2 bis 4 bedeutet,

B für n-wertige Reste der Strukturen

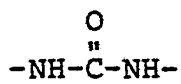 , 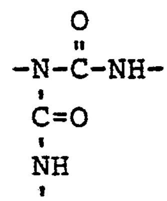 ,

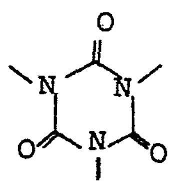 , 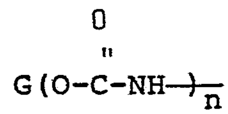 ;

G für einen Rest, wie er durch Entfernen von n OH-Gruppen aus einer n-wertigen Polyhydroxylverbindung des Molekulargewichts 62-10 000 erhalten wird, und

A für einen aromatischen Rest der allgemeinen Formel

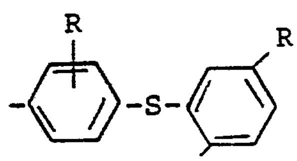

oder einen araliphatischen Rest der allgemeinen Formel

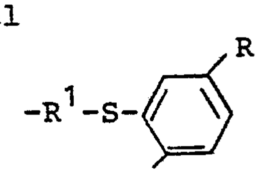

worin

R einen Alkyl- und/oder Alkylthiorest mit 1 bis 4 Kohlenstoffatomen und

$R^1$ einen linearen oder verzweigten aliphatischen Kohlenwasserstoffrest mit 2 bis 4 Kohlenstoffatomen bedeuten, stehen,

verwendet.

3) Verfahren nach Anspruch 2, dadurch gekennzeichnet, daß als NCO-Voraddukt ein Umsetzungsprodukt aus einem zwei oder drei Hydroxylgruppen aufweisenden Polyether mit einem Molekulargewicht von 400 bis 6000 sowie gegebenenfalls einem Polyol mit einem Molekulargewicht zwischen 62 und 400 und einem schwefelhaltigen Polyisocyanat im NCO/OH-Verhältnis von 1,5:1 bis 2,8:1 eingesetzt wird.

4) Verfahren nach Anspruch 2, dadurch gekennzeichnet, daß als NCO-Voraddukt ein Umsetzungsprodukt aus einer OH-Verbindung mit einem Molekulargewicht zwischen 18 und 400 und einem schwefelhaltigen Polyisocyanat im NCO/OH-Verhältnis von 1,5:1 bis 2,8:1 eingesetzt wird.

5) Verfahren nach Ansprüchen 2 bis 4, dadurch gekennzeichnet, daß im ersten Verfahrensschritt ein Äquivalentverhältnis zwischen Hydroxylionen und NCO-Gruppen von 1,01:1 bis 1,60:1 eingehalten und daß das NCO-Voraddukt in Form einer Lösung in einem mit Wasser mischbaren inerten organischen Lösungsmittel eingesetzt wird.

6) Verfahren nach Ansprüchen 2 bis 5, dadurch gekennzeichnet, daß Alkalihydroxide in Form einer 5 bis 20 gew.-%igen Lösung eingesetzt werden.

7) Verfahren nach Ansprüchen 2 bis 6, dadurch gekennzeichnet, daß man
   a) das Carbamat in einem zweiten Schritt durch Zusatz einer überschüssigen Menge eines sauren Ionenaustauschers unter Abspaltung von Kohlendioxid in das freie Amin überführt;
   b) das so erhaltene Polyamin aus dem Reaktionsprodukt auf an sich bekannte Weise abtrennt.

8) Verfahren nach Anspruch 7, dadurch gekennzeichnet, daß man pro Alkaliäquivalent 1,01 bis 2 Äquivalente von dem Ionenaustauscherharz entstammende Wasserstoffionen einsetzt.

9) Verfahren nach Anspruch 2, dadurch gekennzeichnet, daß man
   a) das Carbamat in einem zweiten Schritt durch Zusatz einer überschüssigen Menge an Mineralsäure oder starker organischer Säure unter Abspaltung von Kohlendioxid in das Ammoniumsalz überführt;
   b) die überschüssige Mineralsäure oder starke organische Säure durch Basenzusatz neutralisiert und
   c) das so erhaltene Polyamin aus dem Reaktionsprodukt auf an sich bekannte Weise abtrennt.

10) Verwendung der gemäß Ansprüchen 2 bis 9 hergestellten Polyamine als Ausgangsstoffe zur Herstellung von Polyurethanen.